(19)

**Europäisches Patentamt**
**European Patent Office**
**Office européen des brevets**

(11) **EP 4 220 649 A1**

(12) **EUROPEAN PATENT APPLICATION**

(43) Date of publication:
**02.08.2023 Bulletin 2023/31**

(21) Application number: **23154341.4**

(22) Date of filing: **31.01.2023**

(51) International Patent Classification (IPC):
**G16H 10/20** (2018.01)   **G06N 20/00** (2019.01)

(52) Cooperative Patent Classification (CPC):
**G16H 10/20; G06N 20/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.01.2022 US 202263304844 P**

(71) Applicant: **ZS Associates, Inc.**
**Evanston, IL 60201 (US)**

(72) Inventors:
• **BAGGA, Abhishek**
  **Hitchin (GB)**
• **GROVE, Nicholl**
  **Guildford (GB)**
• **OLAH, Zachary**
  **Evanston, 60201 (US)**
• **CARNEY, Christopher**
  **Boston (US)**

(74) Representative: **Murgitroyd & Company**
**Murgitroyd House**
**165-169 Scotland Street**
**Glasgow G5 8PL (GB)**

(54) **METHODS AND APPARATUS FOR MACHINE LEARNING TO CALCULATE A PATIENT BURDEN SCORE FOR PARTICIPATION IN A CLINICAL TRIAL**

(57)     Disclosed herein are methods and systems to predict and quantify a patient's burden when participating in a clinical trial. A method includes gathering data associated with pervious participants and their burden and experiences when participating in clinical trials. The method also includes executing data clean-up protocols to quantify and standardize the previous participants' ex-periences and burden. The method then includes training one or more computer models to identify connections between participants and their unique attributes in light of their standardized burden and to predict a patient burden score for a new patient participating in a new clinical trial.

**FIG. 3**

**Description**

**CROSS REFERENCE TO RELATED APPLICATIONS**

This application claims the benefit of and priority to US Provisional Patent Application No. 63/304844, filed January 31, 2022, which is incorporated herein by reference in its entirety for all purposes.

**TECHNICAL FIELD**

This application relates generally to generating, training, calibrating, and executing computer models to quantify and predict a patient burden score and to populate graphical user interfaces accordingly.

**BACKGROUND**

Forecasting patient burden is conventionally a very slow, expensive, inefficient, and inaccurate process. Conventionally, a team of human analysts requests feedback from a representative sample of patients who have participated in clinical trials. The human analysis may then use their subjective skills and understanding (and sometimes conventional computer-implemented methods, such as spreadsheets) to determine a burden associated with the clinical trial. Not only is this process tedious, time-consuming, and expensive, it is also unreliable because the results depend directly on the human reviewer's subjective skills and understanding.

During the past several years, drug developers in the public and private sectors have expressed keen interest in the systematic measurement of participation burden in clinical trials. The demand to quantify participation burden is due to a number of factors including rising investment in patient-centric development planning and execution and the intensifying efforts to manage the ongoing adverse impact of protocol complexity on clinical trial timeliness, efficiency, and cost.

**SUMMARY**

For the aforementioned reasons, there is a need to remove the subjectivity of the conventional approaches and to intelligently and empirically measure the patient burden of a clinical trial. There is a need to develop computer models that can quantify and predict patient burden scores, in a manner that is more accurate than conventional methods.

As used herein, a patient burden is a standard, quantified metric used across clinical development decision-making to inform patient-focused trial design, predict trial performance, and improve the trial patient experience. The methods and systems described herein predict the patient burden to understand the patient experience during an actual or simulated clinical trial and to establish a link between patient burden and clinical trial performance outcomes to accelerate the adoption of patient-focused clinical trials (e.g., drug development) across the industry.

Using the methods and systems discussed herein, a patient burden calculation system can utilize one or more computer models that implement a comprehensive participant burden algorithm based on protocol procedures, participation requirements, and lifestyle preferences of patients/participants to predict a burden score for patients and participants of a clinical trial. The system can survey data associated with various clinical trials and can analyze and generate the algorithm accordingly. Specifically, the system can train artificial intelligence (AI) and machine learning (ML) models to uncover hidden patterns and make connections between a participant's burden and various operational parameters of a clinical trial, which were previously impractical and not feasible using conventional methods.

Using the methods and systems disclosed herein, the system can also perform descriptive statistics, significance tests, and univariate analyses to ensure the accuracy and fitness of the model. Using the methods and systems described herein, strong statistically significant associations can be established between the participant burden algorithm and protocol performance outcomes including cycle times, number of amendments, and screen failure rates.

The present disclosure presents an advancement in computer modeling and empirical analysis of quantifying participation burden that will assist clinicians (e.g., drug development teams) and protocol authors in retrospectively understanding clinical trial performance outcomes and in prospectively informing protocol design decisions.

In an embodiment, a method comprises in response to transmitting a clinical study questionnaire to a set of patients associated with a set of clinical studies, retrieving, by a processor, input received via the set of patients, the input corresponding to demographic data and a quantified burden associated with each clinical study; generating, by the processor, a training dataset comprising: each patient's demographic data, a patient burden score for each patient generated in accordance with an algorithm evaluating each patient's input with regards to participation logistics, lifestyle factors, caregiver involvement, and procedural burden associated with each clinical study, and a set of operational parameters associated with the set of clinical study; and training, by the processor, a computer model using the training dataset, such that the computer model is configured to ingest data associated with a new clinical study and predict a new patient burden score.

The new patient burden score may be further dependent on an attribute of the new patient.

The method may further comprise populating, by the processor, at least one graphical user interface using the new patient burden score.

The set of operational parameters may comprise at least one of medications, lab tests, blood tests, examinations, non-invasive procedures, invasive procedure, imaging procedure burden, or self-assessment questionnaire burden.

The trained computer model may identify an estimated elasticity for a relationship between strength of features within the training dataset.

The training may comprise using an iterative multivariate elimination regression modeling protocol to determine which input has a statistically significant relationship with the patient burden score.

The training dataset may be labeled and the computer model is trained via a supervised training method.

In another embodiment, a system comprises a server comprising a processor and a non-transitory computer-readable medium containing instructions that when executed by the processor causes the processor to perform operations comprising: in response to transmitting a clinical study questionnaire to a set of patients associated with a set of clinical studies, retrieve input received via the set of patients, the input corresponding to demographic data and a quantified burden associated with each clinical study; generate a training dataset comprising: each patient's demographic data, a patient burden score for each patient generated in accordance with an algorithm evaluating each patient's input with regards to participation logistics, lifestyle factors, caregiver involvement, and procedural burden associated with each clinical study, and a set of operational parameters associated with the set of clinical study; and train a computer model using the training dataset, such that the computer model is configured to ingest data associated with a new clinical study and predict a new patient burden score.

The new patient burden score may be further dependent on an attribute of the new patient.

The instructions may further cause the processor to: populate at least one graphical user interface using the new patient burden score.

The set of operational parameters may comprise at least one of medications, lab tests, blood tests, examinations, non-invasive procedures, invasive procedure, imaging procedure burden, or self-assessment questionnaire burden.

The trained computer model may identify an estimated elasticity for a relationship between strength of features within the training dataset.

The training may comprise using an iterative multivariate elimination regression modeling protocol to determine which input has a statistically significant relationship with the patient burden score.

The training dataset may be labeled and the computer model is trained via a supervised training method.

In another embodiment, a system comprises a server configured to: in response to transmitting a clinical study questionnaire to a set of patients associated with a set of clinical studies, retrieve input received via the set of patients, the input corresponding to demographic data and a quantified burden associated with each clinical study; generate a training dataset comprising each patient's demographic data, a patient burden score for each patient generated in accordance with an algorithm evaluating each patient's input with regards to participation logistics, lifestyle factors, caregiver involvement, and procedural burden associated with each clinical study, and a set of operational parameters associated with the set of clinical study; and train a computer model using the training dataset, such that the computer model is configured to ingest data associated with a new clinical study and predict a new patient burden score.

The new patient burden score may be further dependent on an attribute of the new patient.

The server may be further configured to populate at least one graphical user interface using the new patient burden score.

The set of operational parameters may comprise at least one of medications, lab tests, blood tests, examinations, non-invasive procedures, invasive procedure, imaging procedure burden, or self-assessment questionnaire burden.

The trained computer model may identify an estimated elasticity for a relationship between strength of features within the training dataset.

The training may comprise using an iterative multivariate elimination regression modeling protocol to determine which input has a statistically significant relationship with the patient burden score.

The data associated with the set of clinical studies may correspond to at least one operational parameter of each clinical study.

## BRIEF DESCRIPTION OF THE DRAWING FIGURES

Objects, aspects, features, and advantages of embodiments disclosed herein will become more fully apparent from the following detailed description, the appended claims, and the accompanying drawing figures in which reference numerals identify similar or identical elements. Reference numerals that are introduced in the specification in association with a drawing figure may be repeated in one or more subsequent figures without additional description in the specification to provide context for other features, and not every element may be labeled in every figure. The drawing figures are not necessarily to scale, emphasis instead being placed upon illustrating embodiments, principles, and concepts. The drawings are not intended to limit the scope of the claims included herewith.

**FIG. 1A** is a block diagram of embodiments of a computing device;

**FIG. 1B** is a block diagram depicting a computing environment comprising client devices in communication with cloud service providers;

**FIG. 2** is a block diagram of an example system in which performance prediction management services may manage and streamline access by clients to resource feeds (via one or more gateway services) and/or software-as-a-service (SaaS) applications;

**FIG. 3** is an example computing environment for the patient burden calculation system, in accordance with one or more implementations; and

FIGS. 4A-F illustrate examples of workflow executed by the patient burden calculation system, in accordance with one or more implementations.

The features and advantages of the present solution will become more apparent from the detailed description set forth below when taken in conjunction with the drawings, in which like reference characters identify corresponding elements throughout. In the drawings, reference numbers generally indicate identical, functionally similar, and/or structurally similar elements.

## DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used and/or other changes may be made without departing from the spirit or scope of the present disclosure. The illustrative embodiments described in the detailed description are not meant to be limiting of the subject matter presented.

Section A describes a computing environment that may be useful for practicing embodiments described herein;

Section B describes a non-limiting example of a patient burden calculation system; and

Section C describes non-limiting examples of methods to develop and implemented an algorithm to predict a patient burden score.

Section A: Computing Environment:

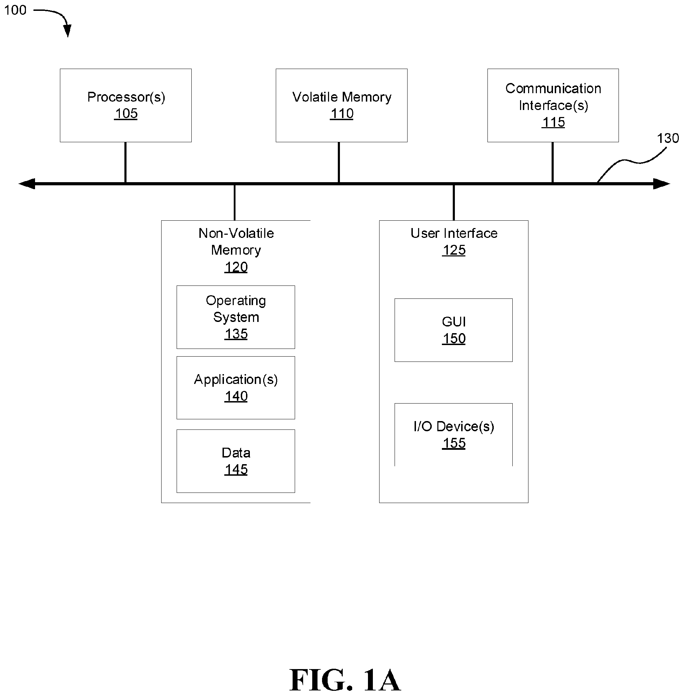

Prior to discussing the specifics of embodiments of the systems and methods of an appliance and/or client, it may be helpful to discuss the computing environments in which such embodiments may be deployed.
As shown in **FIG. 1A,** computer **100** may include one or more processors **105,** volatile memory **110** (e.g., random access memory (RAM)), non-volatile memory **120** (e.g., one or more hard disk drives (HDDs) or other magnetic or optical storage media, one or more solid-state drives (SSDs) such as a flash drive or other solid-state storage media, one or more hybrid magnetic and solid-state drives, and/or one or more virtual storage volumes, such as cloud storage, or a combination of such physical storage volumes and virtual storage volumes or arrays thereof), user interface (UI) **125,** one or more communications interfaces **115,** and communication bus **130.** User interface **125** may include a graphical user interface (GUI) **150** (e.g., a touchscreen, a display, etc.) and one or more input/output (I/O) devices **155** (e.g., a mouse, a keyboard, a microphone, one or more speakers, one or more cameras, one or more biometric scanners, one or more environmental sensors, one or more accelerometers, etc.). The non-volatile memory **120** stores operating system **135,** one or more applications **140,** and data **145** such that, for example, computer instructions of operating system **135** and/or applications **140** are executed by processor(s) **105** out of volatile memory **110.** In some embodiments, volatile memory **110** may include one or more types of RAM and/or a cache memory that may offer a faster response time than the main memory. Data may be entered using an input device of GUI **150** or received from I/O device(s) **155.**

Various elements of computer **100** may communicate via one or more communication buses, shown as communication bus **130.**

Computer **100** as shown in **FIG. 1A** is shown merely as an example, as clients, servers, intermediary, and other networking devices and may be implemented by any computing or processing environment and with any type of machine or set of machines that may have suitable hardware and/or software capable of operating as described herein. Processor(s) **105** may be implemented by one or more programmable processors to execute one or more executable instructions, such as a computer program, to perform the functions of the system. As used herein, the term "processor" describes circuitry that performs a function, an operation, or a sequence of operations. The function, operation, or sequence of operations may be hardcoded into the circuitry or soft coded by way of instructions held in a memory device and executed by the circuitry.

A "processor" may perform the function, operation, or sequence of operations using digital values and/or using analog signals. In some embodiments, the "processor" can be embodied in one or more application-specific integrated circuits (ASICs), microprocessors, digital signal processors (DSPs), graphics processing units (GPUs), microcontrollers, field-programmable gate arrays (FPGAs), programmable logic arrays (PLAs), multi-core processors, or general-purpose computers with associated memory. The "processor" may be analog, digital or mixed-signal. In some embodiments, the "processor" may be one or more physical processors or one or more "virtual" (e.g., remotely located or "cloud") processors. A processor including multiple processor cores and/or multiple processors may provide functionality for parallel, simultaneous execution of instructions, or for parallel, simultaneous execution of one instruction on more than one piece of data.

Communications interfaces **115** may include one or more interfaces to enable computer **100** to access a computer network such as a Local Area Network (LAN), a Wide Area Network (WAN), a Personal Area Network (PAN), or the Internet through a variety of wired and/or wireless or cellular connections.

In described embodiments, the computing device **100** may execute an application on behalf of a user of a client computing device. For example, the computing device 100 may execute a virtual machine, which provides an execution session within which applications execute on behalf of a user or a client computing device, such as a hosted desktop session. The computing device **100** may also execute a terminal services session to provide a hosted desktop environment. The computing device **100** may provide access to a computing environment including one or more of one or more applications, one or more desktop applications, and one or more desktop sessions in which one or more applications may execute.

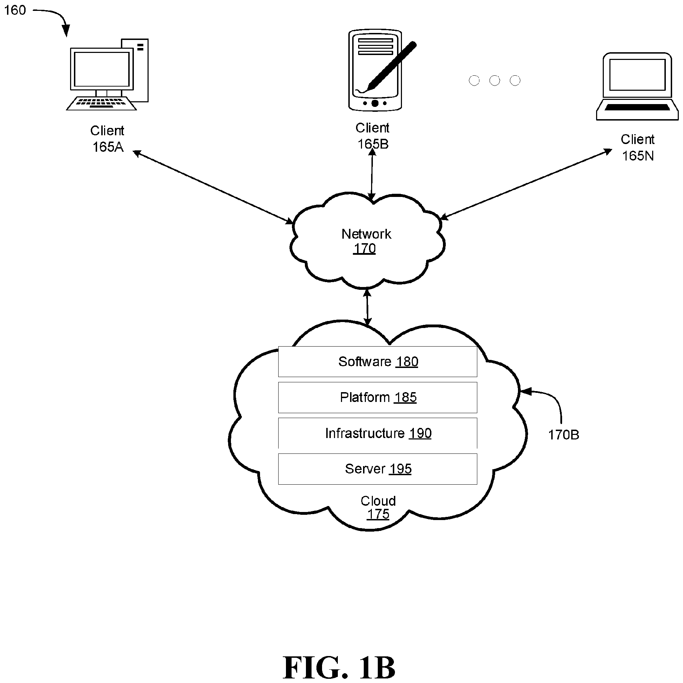

Referring to **FIG. 1B,** a computing environment **160** is depicted. Computing environment **160** may generally be implemented as a cloud computing environment, an on-premises ("on-prem") computing environment, or a hybrid computing environment including one or more on-prem computing environments and one or more cloud computing environments. When implemented as a cloud computing environment, also referred to as a cloud environment, cloud computing, or cloud network, computing environment **160** can provide the delivery of shared services (e.g., computer services) and shared resources (e.g., computer resources) to multiple users. For example, the computing environment **160** can include an environment or system for providing or delivering access to a plurality of shared services and resources to a plurality of users through the internet. The shared resources and services can include but are not limited to, networks, network bandwidth, servers, processing, memory, storage, applications, virtual machines, databases, software, hardware, analytics, and intelligence.

In some embodiments, the computing environment **160** may provide client **165** with one or more resources provided by a network environment. The computing environment **160** may include one or more clients **165a-165n,** in communication with a cloud **175** over one or more networks **170.** Clients **165** may include, e.g., thick clients, thin clients, and zero clients. The cloud **108** may include back-end platforms, e.g., servers, storage, server farms, or data centers. The clients **165** can be the same as or substantially similar to computer **100** of **FIG. 1A.**

The users or clients **165** can correspond to a single organization or multiple organizations. For example, the computing environment **160** can include a private cloud serving a single organization (e.g., enterprise cloud). The computing environment **160** can include a community cloud or public cloud serving multiple organizations. In some embodiments, the computing environment **160** can include a hybrid cloud that is a combination of a public cloud and a private cloud. For example, the cloud **175** may be public, private, or hybrid. Public clouds **108** may include public servers that are maintained by third parties to the clients **165** or the owners of the clients **165.** The servers may be located off-site in remote geographical locations as disclosed above or otherwise. Public clouds **175** may be connected to the servers over a public network **170.** Private clouds **175** may include private servers that are physically maintained by clients **165** or owners of clients **165.** Private clouds **175** may be connected to the servers over a private network **170.** Hybrid clouds **175** may include both the private and public networks **170** and servers.

The cloud **175** may include back-end platforms, e.g., servers, storage, server farms, or data centers. For example, the cloud **175** can include or correspond to a server or system remote from one or more clients **165** to provide third-party control over a pool of shared services and resources. The computing environment **160** can provide resource pooling to serve multiple users via clients **165** through a multi-tenant environment or multi-tenant model with different physical and virtual resources dynamically assigned and reassigned responsive to different demands within the respective environment. The multi-tenant environment can include a system or architecture that can provide a single instance of the software, an application, or a software application to serve multiple users. In some embodiments, the computing environment **160** can provide on-demand self-service to unilaterally provision computing capabilities (e.g., server time, network storage) across a network for multiple clients 165. The computing environment **160** can provide elasticity to dynamically scale out or scale in responsive to different demands from one or more clients **165.** In some embodiments, the computing environment **160** can include or provide monitoring services to monitor, control, and/or generate reports corresponding to the provided shared services and resources.

In some embodiments, the computing environment **160** can include and provide different types of cloud computing services. For example, the computing environment **160** can include Infrastructure as a service (IaaS). The computing environment **160** can include Platform as a service (PaaS). The computing environment **160** can include serverless computing. The computing environment **160** can include Software as a service (SaaS). For example, the cloud **175** may also include a cloud-based delivery, e.g., Software as a Service (SaaS) **180,** Platform as a Service (PaaS) **185,** and Infrastructure as a Service (IaaS) 190. IaaS may refer to a user renting the use of infrastructure resources that are needed during a specified time period. IaaS providers may offer storage, networking, servers, or virtualization resources from large pools, allowing the users to quickly scale up by accessing more resources as needed. Examples of IaaS include AMAZON WEB SERVICES provided by Amazon.com, Inc., of Seattle, Washington; RACKSPACE CLOUD provided by Rackspace US, Inc., of San Antonio, Texas; Google Compute Engine provided by Google Inc. of Mountain View, California; or RIGHTSCALE provided by RightScale, Inc., of Santa Barbara, California. PaaS providers may offer functionality provided by IaaS, including, e.g., storage, networking, servers, or virtualization, as well as additional resources such as, e.g., the operating system, middleware, or runtime resources. Examples of PaaS include WINDOWS AZURE provided by Microsoft Corporation of Redmond, Washington; Google App Engine provided by Google Inc.; and HEROKU provided by Heroku, Inc., of San Francisco, California. SaaS providers may offer the resources that PaaS provides, including storage, networking, servers, virtualization, operating system, middleware, or runtime resources. In some embodiments, SaaS providers may offer additional resources including, e.g., data and application resources. Examples of SaaS include GOOGLE APPS provided by Google Inc.; SALESFORCE provided by Salesforce.com Inc. of San Francisco, California; or OFFICE 365 provided by Microsoft Corporation. Examples of SaaS may also include data storage providers, e.g., DROPBOX provided by Dropbox, Inc., of San Francisco, California; Microsoft SKYDRIVE provided by Microsoft Corporation; Google Drive provided by Google Inc.; or Apple ICLOUD provided by Apple Inc. of Cupertino, California.

Clients **165** may access IaaS resources with one or more IaaS standards, including, e.g., Amazon Elastic Compute Cloud (EC2), Open Cloud Computing Interface (OCCI), Cloud Infrastructure Management Interface (CIMI), or OpenStack standards. Some IaaS standards may allow clients access to resources over HTTP and may use Representational State Transfer (REST) protocol or Simple Object Access Protocol (SOAP). Clients **165** may access PaaS resources with different PaaS interfaces. Some PaaS interfaces use HTTP packages, standard Java APIs, JavaMail API, Java Data Objects (JDO), Java Persistence API (JPA), Python APIs, web integration APIs for different programming languages including, e.g., Rack for Ruby, WSGI for Python, or PSGI for Perl, or other APIs that may be built on REST, HTTP, XML, or other protocols. Clients **165** may access SaaS resources through the use of web-based user interfaces, provided by a web browser (e.g., GOOGLE CHROME, Microsoft INTERNET EXPLORER, or Mozilla Firefox provided by Mozilla Foundation of Mountain View, California). Clients **165** may also access SaaS resources through smartphone or tablet applications, including, e.g., Salesforce Sales Cloud or Google Drive app. Clients **165** may also access SaaS resources through the client operating system, including, e.g., Windows file system for DROPBOX.

In some embodiments, access to IaaS, PaaS, or SaaS resources may be authenticated. For example, a server or authentication server may authenticate a user via security certificates, HTTPS, or API keys. API keys may include various encryption standards such as e.g., Advanced Encryption Standard (AES). Data resources may be sent over Transport Layer Security (TLS) or Secure Sockets Layer (SSL).

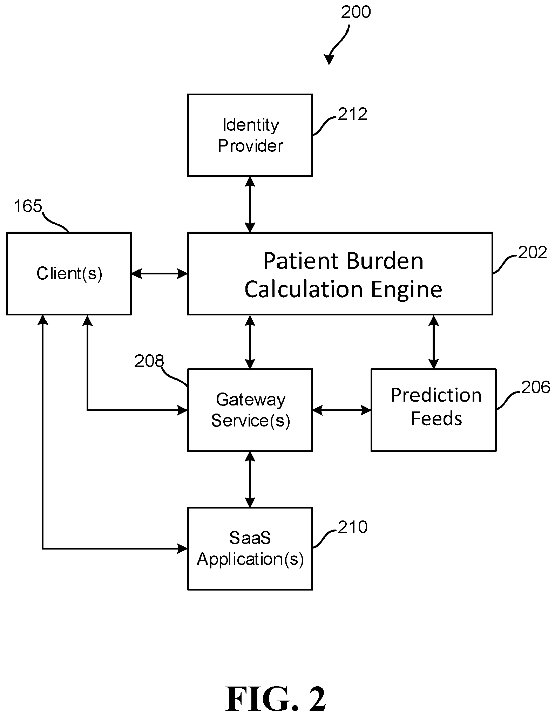

FIG. 2 is a block diagram of an example system **200** in which an a patient burden calculation engine **202** may manage and streamline access by one or more clients **165** to one or more prediction feeds **206** (via one or more gateway services **208)** and/or one or more software-as-a-service (SaaS) applications **210.** As used herein, a prediction feed is a result of the execution of one or more AI models discussed herein. In particular, the patient burden calculation engine **202** may employ an identity provider **212** to authenticate the identity of a user of a client **165** and, following authentication, identify one or more prediction feeds the user is authorized to access. For the prediction feed(s) **206,** the client **165** may input attributes associated with a clinical trial and may request access to one or more AI models via a gateway service **208.** For the SaaS application(s) **210,** the client **165** may access the selected application directly. The SaaS application(s) **210** may allow the client **165** to access the platform discussed herein and view the prediction feeds **206.**

The client(s) **165** may be any type of computing device capable of accessing the prediction feed(s) **206** and/or the SaaS application(s) **210,** and may, for example, include a variety of desktop or laptop computers, smartphones, tablets, etc. Each of the patient burden calculation engine **202,** the prediction feed(s) **206,** the gateway service(s) **208,** the SaaS application(s) **210,** and the identity provider **212** may be located within an on-premises data center of an organization for which the system **200** is deployed, within one or more cloud computing environments, or elsewhere.

Section B: Patient Burden Calculation System

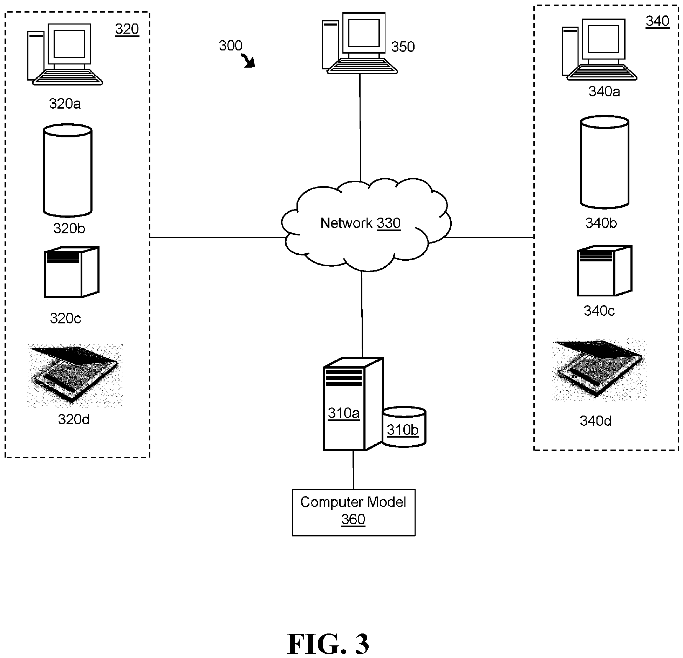

As will be described throughout, a server of patient burden calculation system **300** (such as an analytics server **310a**) can retrieve and analyze data using various methods described herein to calculate a patient burden score and present the calculations in a manner that is easily consumable and customizable for end-users. **FIG. 3** is a non-limiting example of components of the patient burden calculation system **300** in which the analytics server **310a** operates. The analytics server may be any computer, server, or processor described in **FIGS. 1A-2.**

The analytics server **310a** may utilize features described in **FIG. 3** to retrieve data and to generate/display results. The analytics server **310a** is communicatively coupled to a system database **310b**, electronic data sources **320a-d** (collectively electronic data sources **320**), end-user devices **340a-d** (collectively end-user device **340**), and an administrator computing device **350**. The system **300** is not confined to the components described herein and may include additional or alternative components, not shown for brevity, which is to be considered within the scope of the embodiments described herein.

The above-mentioned components may be connected through a network **330**. The examples of the network **330** may include but are not limited to, private or public LAN, WLAN, MAN, WAN, and the Internet. The network **330** may include both wired and wireless communications according to one or more standards and/or via one or more transport mediums.

The analytics server **310a** may utilize one or more application programming interfaces (APIs) to communicate with one or more of the electronic devices described herein. For instance, the analytics server may utilize APIs to automatically receive data from the electronic data sources **320**. The analytics server **310a** can receive data as it is generated, monitored, and/or processed by the electronic data source **320**. For instance, the analytics server **110a** may utilize an API to receive clinical trial data from the database **320b** without any human intervention. This automatic communication allows for faster retrieval and processing of data.

The analytics server **310a** may generate and/or host an electronic platform having a series of graphical user interfaces (GUIs) configured to use various computer models to project and display data associated with a clinical trial. The platform can be displayed on the electronic data sources **320,** the administrator computing device **350,** and/or end-user devices **340**. An example of the platform generated and/or hosted by the analytics server **310a** may be a web-based application or a website configured to be displayed on different electronic devices, such as mobile devices, tablets, personal computers, and the like. Even though certain embodiments discuss the analytics server **310a** displaying the results, it is expressly understood that the analytics server **310a** may either directly generate and display the platform described herein or may present the data to be presented on a GUI displayed on the end-user devices **340.**

The analytics server **310a** may host a website (also referred to herein as the platform) accessible to end-users operating any of the electronic devices described herein (e.g., end-users), where the content presented via the various webpages may be controlled based upon each particular user's role or viewing permissions. The analytics server **310a** may be any computing device comprising a processor and non-transitory machine-readable storage capable of executing the various tasks and processes described herein. Non-limiting examples of such computing devices may include servers, computers, workstation computers, personal computers, and the like. While this example of the system **300** includes a single analytics server **310a,** in some configurations, the analytics server **310a** may include any number of computing devices operating in a distributed computing environment.

The analytics server **310a** may execute one or more software applications configured to display the platform (e.g., host a website), which may generate and serve various webpages to each electronic data sources **320** and/or end-user devices **340**. Different end-users may use the website to view and/or interact with the predicted results.

The analytics server **310a** may be configured to require user authentication based upon a set of user authorization credentials (e.g., username, password, biometrics, cryptographic certificate, and the like). In such implementations, the analytics server **310a** may access the system database **310b** configured to store user credentials, which the analytics server **310a** may be configured to reference to determine whether a set of entered credentials (purportedly authenticating the user) match an appropriate set of credentials that identify and authenticate the user.

The analytics server **310a** may also store data associated with each user operating one or more electronic data sources **320** and/or end-user devices **340**. The analytics server **310a** may use the data to determine whether a user device is authorized to view results generated computer model(s) discussed herein, such as the computer model **360**.

The computer model **360** may be any collection of one or more algorithms and machine-readable code that can ingest data associated with a patient and/or a clinical trial and to predict a patient burden score. Accordingly, the computer model **360** may include a mathematical algorithm. Additionally or alternatively, the computer model **360** may represent an AI/ML model (e.g., neural network) that can be trained in accordance with data received from the electronic data sources **320** and/or end-user devices **340**. Specifically, the analytics server **310** may use the data collected from the electronic data sources **320** to generate a training dataset and further train the AI model **360** using various machine learning techniques (e.g., supervised, unsupervised, or semi-supervised).

The analytics server **310a** may receive data associated with a clinical trial from end-user devices **340** and/or electronic data sources **320**. The electronic data sources **320** may represent different databases or third-party vendors who possess medical data, marketing data, clinical trial data, and the like. For instance, the electronic data sources **320** may represent computers, databases, and servers of a medical provider that can provide additional information regarding a clinical trial.

The analytics server **310a** may use the data collected from the electronic data sources **320** and received from the end-user device **340** to execute the computer model **360**. The analytics server **310a** then displays the results via the platform (e.g., GUIs) on the administrator computing device **350** or the end-user devices **340.**

The end-user devices **340** may be any computing device comprising a processor and a non-transitory machine-readable storage medium capable of performing the various tasks and processes described herein. Non-limiting examples of an end-user device may include workstation computers, laptop computers, tablet computers, and server computers. In operation, various end-users may use end-user devices **340** to access the platform operationally managed by the analytics server **310a** to enter clinical trial information and view predicted/projected results.

The administrator computing device **350** may represent a computing device operated by a system administrator. The administrator computing device **350** may be configured to display retrieved data, in the form of results generated by the analytics server **110a,** where the system administrator can monitor various models utilized by the analytics server **110a,** review feedback, and modify various thresholds/rules described herein.

The analytics server **310a** may access, generate, and execute various computer models. Although the example system **300** depicts the computer model **360** stored on the analytics server **310a,** the AI models may be stored on another device or server (e.g., store locally or in cloud storage).

In operation, the analytics server **310a** may collect data associated with various clinical trials (e.g., operational parameters associated with the clinical trials) and other patient experiences from a variety of sources (e.g., from the electronic data sources **320** or from the patients directly via a global survey). The analytics server **310a** may then train the computer model **360** to develop an algorithm to calculate/predict a patient burden score. When the computer model **360** is trained, the analytics server **310** may implement the computer model, and allow end-users to use the computer model **360** to view results. For instance, an end-user may use any of the end-user devices **340** to access the platform and enter attributes of a clinical trial. The analytics server **310a** may then execute the computer model **360** to calculate a patient burden score and may populate one or more GUIs to display the results.

Section C: non-limiting examples of methods to develop and implement an algorithm to predict a patient burden score:

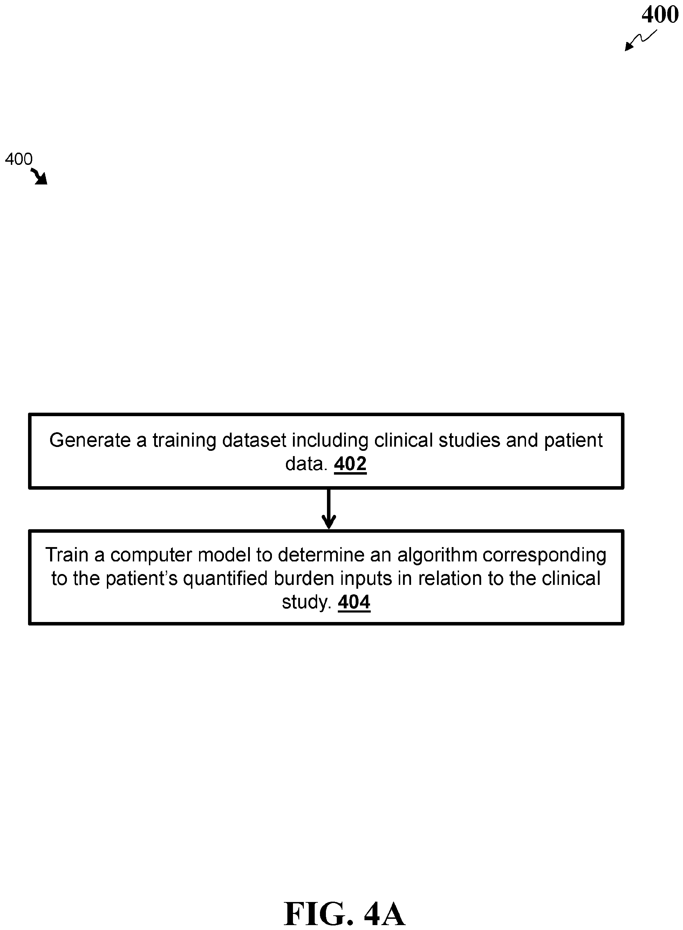

Referring now to **FIG. 4A,** a workflow diagram for patient burden calculation system is depicted, in accordance with one or more implementations. The method **400** includes steps **402-404.** However, other embodiments may include additional or alternative execution steps or may omit one or more steps altogether. In addition, the method **400** is described as being executed by a system, similar to the system described in **FIG. 3.** Different steps of the method **400** or different parts of the different steps may be executed by any number of computing devices operating in the distributed computing system described in **FIGS. 1A-3.**

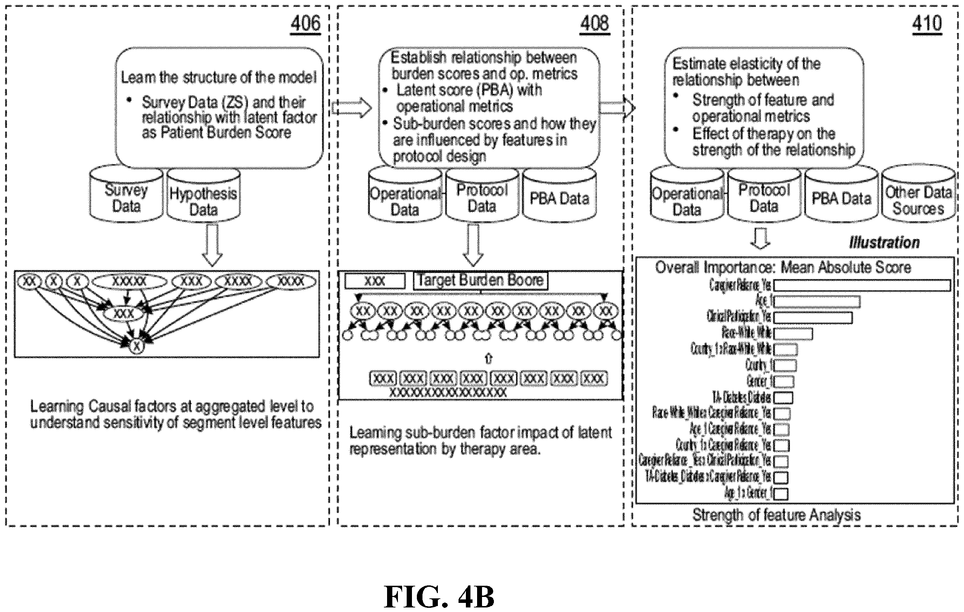

At step **402,** the analytics server may generate a training dataset including clinical study and patient data. Using the method **400,** the analytics server may generate and train a computer model that uses an enhanced baselined algorithm to quantify a patient burden and translate the burden to trial performance (e.g., evaluate new clinical studies). As depicted in **FIG. 4B,** the analytics server may use a three-step process to understand how protocol design and patient attributes influence operational metrics and patient burden.

Specifically, the analytics server may generate and train a model (e.g., computer model **360)** to first learn the structure of the data and develop an algorithm to predict a patient burden score (step **406).** The analytics server may then establish a relationship between burden score and operational metrics (step **408).** For instance, the computer model may learn the sub-burden factor impact of latent representation by therapy area. Finally, at step **410,** the analytics server may examine the features within the data to identify/estimate an elasticity of the relationship between the strength of features and operational metrics and effects of therapy on the strength of the relationship.

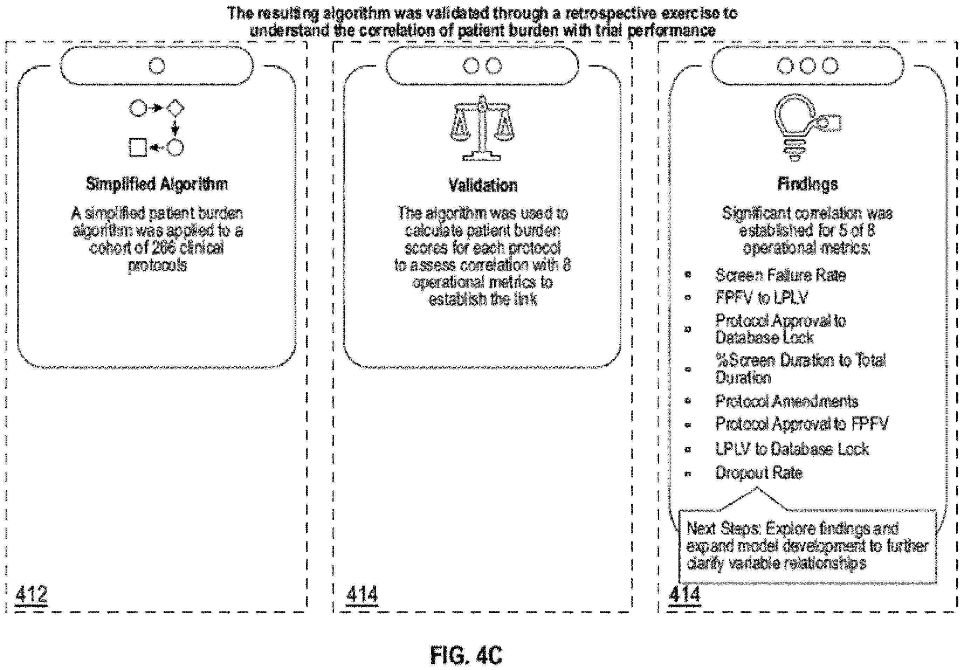

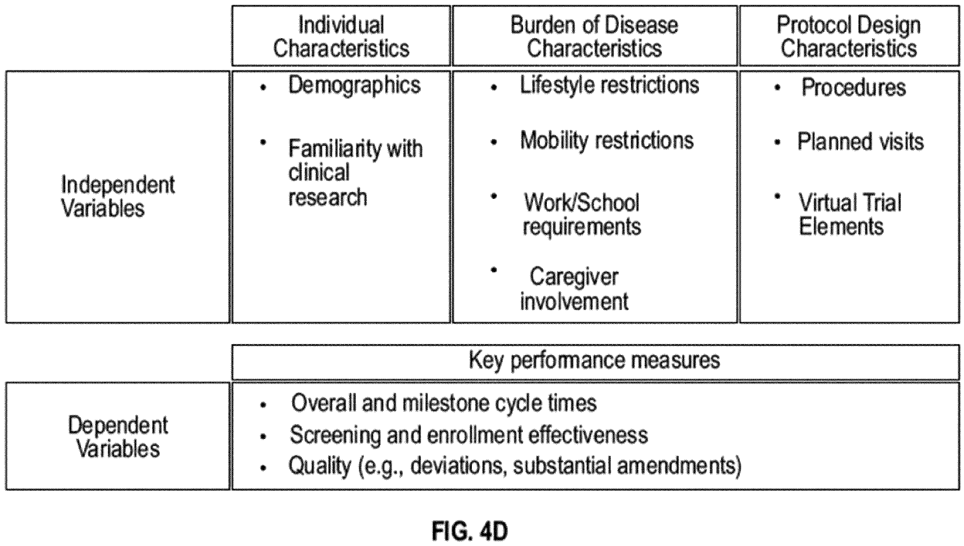

To develop the algorithm itself (e.g., to train the computer model), the analytics server may use a two-step process depicted in **FIG. 4C.** The analytics server may first analyze data to generate the algorithm **(412).** When developing the algorithm, the analytics server may use a variety of factors (independent and dependent variables), as depicted in **FIG. 4D.** Referring back to **FIG. 4C,** the analytics server may then test and validate the algorithm **(414).** Specifically, the analytics server may calculate a patient burden score for each protocol to assess correlation with different operational metrics and to determine if any connections among the data points exist. As a result, the analytics server may establish various connections and uncover patterns of how operational parameters of a clinical trial relate to patient burden and experiences as indicated by patient's responses and inputs.

To train the computer model and develop an algorithm that can predict the patient burden, the analytics server may first generate data associated with participants of previous clinical trials (e.g., training dataset). The analytics server may use the training dataset to develop the algorithm.

In order to retrieve pertinent data and to generate the training dataset, the analytics server may implement a global study and collect data associated with past patients and participants of a clinical trial. For instance, the analytics server may transmit interactive questionnaires to various computing devices associated with past participants of different clinical trials (e.g., electronic data sources **320** discussed in **FIG. 3).** In some configurations, an administrator (operating the administrator computer **350)** may conduct a large global online survey of individuals with and/or without prior clinical research participation experience. The analytics server may then aggregate, de-duplicate, analyze, and compile the perceived burden of procedures and participation conditions into scores, sensitive to variation by participant subgroup(s), thereby generating the training dataset. The analytics server may generate an algorithm accordingly and store the algorithm within a computer model (e.g., computer model **360).** The analytics server may also test and validate the algorithm and its ability to predict protocol performance outcomes.

The survey transmitted to the participants may incorporate questions that probe lifestyle factors, medication adherence burden, transportation and distance requirements, and remote and/or virtual study visit approaches. The analytics server may divide the survey instrument into five core sections: demographics, participation logistics, lifestyle factors, caregiver involvement, and procedural burden. The analytics server may analyze each category individually in order to identify an algorithm that can assess a patient's needs and predict a score indicating their burden in participating in a clinical trial.

The survey may include various input elements designed to receive input from the participants. For instance, the perceived procedural burden may be assessed using the numerical scale (e.g., from 0-100) and a corresponding input element allowing the participants to input their responses. For this scale, respondents may be asked to compare the perceived procedural burden relative to the administration of procedures to gather vital signs (e.g., temperature, blood pressure, and pulse) in order to standardize scores across cultures and individual tolerances.

Once the data is received, the analytics server may aggregate the data into one or more training datasets. Referring back to **FIG. 4A,** at step **404,** the analytics server may then train a computer model to generate an algorithm corresponding to the patient's quantified burden inputs. The algorithm, as used herein, may learn from the training dataset and may uncover patterns and links among data points within the training dataset to identify how a patient's experiences (e.g., indicated by the responses received from the patients) relate to the clinical study/trial.

The algorithm may derive protocol-specific dimensions associated with patient burden to analyze the data, such as procedural (e.g., effort, time commitment, anxiety and pain associated with each procedure), convenience (e.g., number of visits, travel distance, type of transportation, days of work missed, or arranging for child care), lifestyle (e.g., restrictions associated with diet, exercise, smoking, or alcohol consumption), caregiver involvement (e.g., enrolling in the study, recording data and notes, helping administer study drug, or providing transportation and child care), and the like.

When the algorithm is finalized, the computer model may then ingest new data (e.g., data associated with a new clinical study and/or a new patient participating in the new study) and may predict a burden sore for the new clinical study. For instance, the computer model can predict specific performance metrics (corresponding predicted patient burden) regarding screen failure rate, first patient first visit (FPFV) to last patient last visit (LPLV), protocol approval, percentage of screen to total duration, protocol amendments, protocol approval to FPFV, LPLV to database lock, and/or dropout rate.

The computer model may include any algorithm (whether utilizing AI/ML techniques or not). For instance, the computer model may be a mathematical algorithm that is not developed using AI/ML techniques. Additionally or alternatively, the computer model may comprise a neural network that is iteratively trained by the analytics server using the training dataset. In an alternative embodiment, the analytics server may use a gradient boosting method to train the computer model. However, it is expressly understood that the computer model is not limited to either method.

In various non-limiting embodiments, the computer model may use one or more deep learning engines to train itself. Although exemplified using deep neural networks, it should be understood that any alternative and/or additional deep learning model(s) might be used to implement deep learning engines. The deep learning engines include processing pathways that are trained during the training phase. A multi-layer neural network may consist of a stack of layers each performing a specific operation, e.g., pooling, loss calculation, etc. Each intermediate layer receives the output of the previous layer as its input. The beginning layer may be an input layer, which is directly connected to an input received from the training dataset. The next set of layers may be convolutional layers that present the results of convolving a certain number of filters with the input data and perform as a feature extractor. The output of each layer may be considered as an activation map, which highlights the effect of applying a specific analytical protocol on the input.

The analytics server may train the computer model using a supervised method where the data records within the training dataset are labeled. For instance, the training dataset may be labeled, such that the computer model can identify and distinguish which input corresponds to which operational parameter of the clinical study that was reviewed positively (or poorly) by one or more patients.

Additionally or alternatively, the analytics server may use an unsupervised method where the training dataset is not labeled. Because labeling the data within the training dataset may be time-consuming and may require vast computing power, the analytics server may utilize unsupervised training techniques to train the computer model. The analytics server may not be limited to the above-described AI/MI, training techniques. For instance, the analytics server may use both techniques, wherein the analytics server may label data when applicable and use a supervised training method (e.g., certain portions of the data are labeled as ground truth). If the analytics server cannot verify the accuracy of portions of the data retrieved, the analytics server may use an unsupervised training method. Therefore, the analytics server may use a semi-supervised method to train the computer model. Additionally or alternatively, the analytics server may also utilize reinforcement learning to train the computer model.

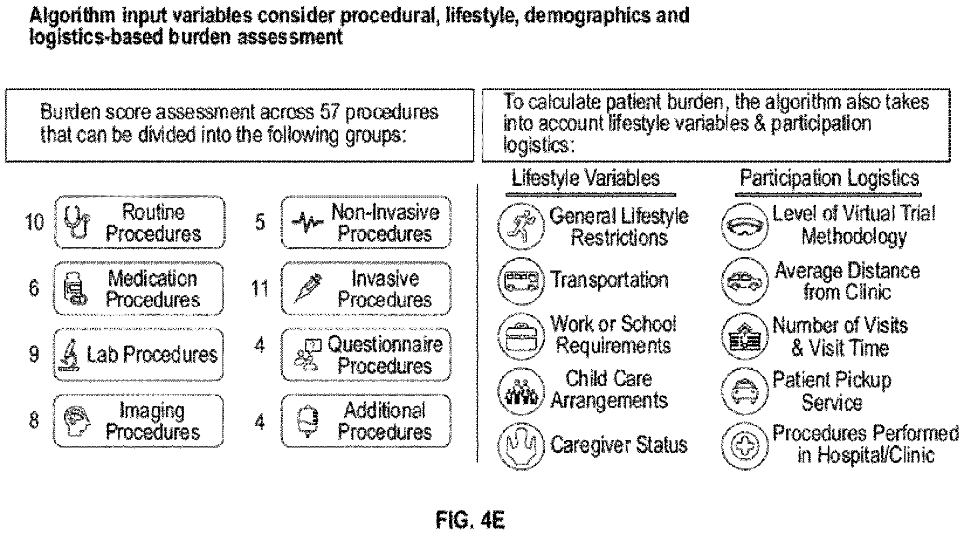

During training, the analytics server may manipulate the data and analyze different portions of the data. For instance, the computer model may use a data split to segment the data into different subgroups. As depicted in **FIG. 4E,** for different common procedures, the analytics server may divide the responses into eight procedure groups:

First: medications (6 procedures);

Second: lab and blood tests (9 procedures);

Third: routine examinations (10 procedures);

Fourth: non-invasive procedures (5 procedures);

Fifth: invasive procedures (11 procedures);

Sixth: imaging procedure burden (8 procedures);

Seventh: self-assessment questionnaire burden (4 procedures); and

Eighth: other common procedures (5 procedures).

Optionally, the analytics server may perform various data cleaning protocols and review the quality of data within the training dataset. For instance, the analytics server may remove the outlier data points that satisfy a predetermined threshold, de-duplicate data points, and the like. In a non-limiting example, responses that met any of the following criteria are evaluated and removed:

Number of disease indications selected $\geq$ 15;

Overall mean procedure burden score < 10;

Overall mean procedure burden score > 90;

Three or more procedure group burden score averages were equal.

These checks can be implemented to filter out those responses received from participants who had consistently provided burden scores that were too low, too high, or too similar.

Next, the analytics server may identify a number of participant subgroups and incorporate the participants into the burden algorithm to be analyzed. The analytics server may utilize various sample size, variance, and linear regression modeling techniques and to analyze and identify those that were most associated with patient burden. Significant differences can be considered if their *correspondingp* values is less than 0.05.

The analytics server may randomly analyze the results to ensure accuracy. For instance, burden for eleven specific participant subgroups may demonstrate the most significant differences. These subgroups may then be incorporated into multivariate regression models to predict each of the eight procedure group burden scores. By eliminating the least significant independent variable (assessed by variable $p$-value) in each iteration until all variables were significant, final predictive models for each procedure group can be established.

The analytics server may also test and validate the algorithm to ensure its accuracy. Specifically, the analytics server may utilize previous studies assessing protocol design practices (e.g., various studies based on convenience samples of actual protocols provided by pharmaceutical, biopharmaceutical, and contract research organizations) to evaluate the model/algorithm's fitness. In a non-limiting example, de-identified protocols compiled by other organizations can be used to test and validate the participation burden algorithm.

Table 1

|  | Overall | Phase I | Phase II | Phase III |
|---|---|---|---|---|
| Total (n) | 266 | 48 | 107 | 111 |
| Top Therapeutic Areas (%) |  |  |  |  |
| Oncology | 35.3% | 56.3% | 37.4% | 24.3% |
| Neurology | 11.3% | 31.3% | 6.5% | 7.2% |
| Infectious Disease | 10.2% | 0% | 15.0% | 9.9% |
| Central Nervous System (CNS) | 9.4% | 2.1% | 10.3% | 11.7% |
| Endocrine | 8.3% | 2.1% | 6.5% | 12.6% |

Table 1 depicts validation performed on the patient burden algorithm. As depicted, in total, a convenience sample of 266 phase I, II, and III protocols from a variety of therapeutic areas were included in a test and validation study.

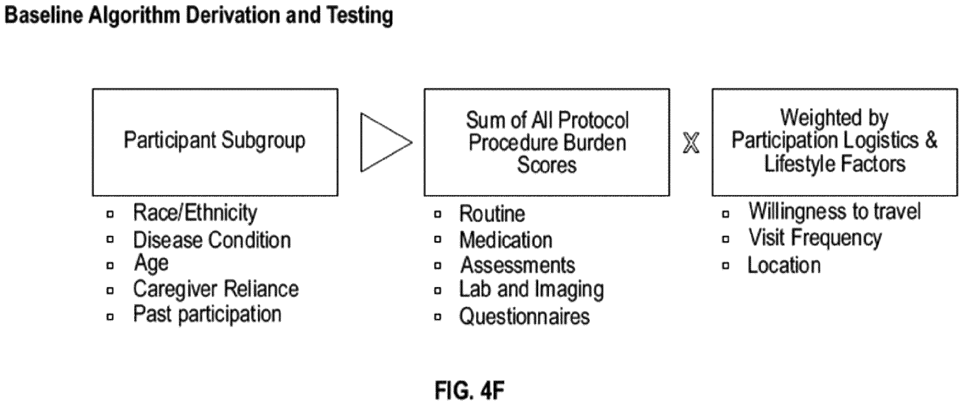

Procedures in each protocol can be assigned to one of the eight procedure groups. These procedure groups can then be assigned average scores based on coefficients calculated in multivariate regression equations. The analytics server may then develop a set of rules to impute which subgroups were active in each protocol in order to demonstrate the flexibility of the algorithm. The analytics server may also use additional rules that were agreed upon to assess participant logistics inflation factors when not available, including the distance patients must travel to the clinic, the number of trial visits, the visit duration, and where procedures were performed. For instance, as depicted in **FIG. 4F,** the analytics server may divide the participants into different subgroups, then apply a formula to identify an initial score for each participant based on data associated with each participant's routine, medication, assessments, labs and imaging, and questionnaires. The analytics server may then weigh the identified burden score by logistics and lifestyle factors (e.g., willingness to travel, visit frequency, location, and the like).

The analytics server may iteratively refine the algorithm until and unless the algorithm satisfies one or more accuracy thresholds. When the algorithm satisfies the accuracy threshold, the computer model can be implemented to analyze new clinical trials and predict a corresponding patient burden score. In a non-limiting example, the below formula represents a simplified patient burden algorithm:

Final Simplified Burden Score = $\sum$

(Number of Medication Procedures * (Medication Coefficient + a + b + c + d + e + f + j + h + I + j + k))

(Number of Lab and Blood Test Procedures * (Lab and Blood Coefficient + a + b + c + d + e + f + j + h + I + j + k))

(Number of Routine Examination Procedures * (Routine Exam Coefficient + a + b + c + d + e + f + j + h + I + j + k))

(Number of Noninvasive Procedures * (Noninvasive Coefficient + a + b + c + d + e + f + j + h + I + j + k))

(Number of Invasive Procedures * (Invasive Coefficient + a + b + c + d + e + f + j + h + I + j + k))

(Number Radiography and Imaging Procedures * (Imaging Coefficient + a + b + c + d + e + f + j + h + I + j + k))

(Number of Questionnaire Procedures * (Questionnaire Coefficient + a + b + c + d + e + f + j + h + I + j + k))

(Number of Additional Procedures * (Additional Coefficient + a + b + c + d + e + f + j + h + I + j + k))

* Average Distance from Clinic Scale
* Number of Visits Scale
* Average Visit Time Scale
* Procedures Performed at Hospital and Clinic Binary

In the above-described formula, variables a-k represent the 11 subgroup coefficient adjustments calculated in multivariate regression models (e.g., coefficients the analytics server incorporates into the equation only when variables were significant predictors of each procedure group mean score).

Because the computer model is iteratively trained, the algorithm can be refined with time, such that the algorithm is iteratively improved. Therefore, the algorithm may not be a static algorithm. That is, certain variables factors used in the algorithm may be refined to fit the data and to produce a more accurate patient burden score.

A wide range of variables can be selected to assess associations with the final burden score for a patient or participant. For instance, variables selected can be screen failure rate, clinical trial cycle time durations (e.g., protocol approval to first patient first visit, first patient first visit to last patient last visit, last patient last visit to database lock, and protocol approval to database lock), dropout rate, protocol amendments, and percent of screening duration to total duration.

Once final algorithm scores for each of the 266 protocols is derived, correlation analyses and linear regression significance testing can be performed for each variable (or at least a portion of the variables). To calculate participation logistics factors, low, medium, and high coefficients can be tested. For instance, low inflation coefficients allow for a maximum inflation rate of 25% for most single variables. Moreover, medium inflation coefficients may double this value to a ceiling of 50% for most variables. Finally, high inflation coefficients may additionally double this value to an upper limit of 100%.

The analytics server may then implement the developed algorithm on a survey collected from actual patients to validate the developed algorithm. In one use case, the analytics server may collect and aggregate survey responses from 2,680 global participants. Following quality checks, 3002 respondents are removed yielding a final global convenience (sample size of n = 269). Survey respondent demographics are summarized in Table 2.

Table 2.

| Total Valid Responses (N) | 3,002 |
| --- | --- |

(continued)

| | Percent Total |
|---|---|
| Distribution by Sex | |
| Male | 47.1% |
| Female | 52.3% |
| Other | 0.6% |
| Distribution by Age | |
| 18-34 | 31.4% |
| 35-44 | 22.9% |
| 45-54 | 16.5% |
| 55-64 | 19.3% |
| ≥65 | 9.9% |
| Distribution by Race | |
| White | 73.5% |
| Black or African American | 9.4% |
| Asian | 15.3% |
| American Indian or Alaskan Native | 1.8% |
| Native Hawaiian or Other Pacific Islander | 0.8% |
| Hispanic/Latino Ethnicity | 17.4% |
| Region | |
| North America | 52.0% |
| Europe | 28.7% |
| Asia | 18.7% |
| South America | 0.5% |
| Africa | 0.1% |
| Therapeutic Areas | |
| Autoimmune | 33.1% |
| Metabolic | 32.3% |
| Central Nervous System | 29.0% |
| Cardiovascular | 25.8% |
| Oncology | 16.1% |
| Has never participated in a clinical trial | 76.8% |

As depicted in Table 2, the distribution of participants (by sex) is roughly equal, with females making up a slightly larger proportion of total responses. Approximately half (52%) of respondents are based in North America; 28.7% in Europe; and 18.7% based in Asia. Nearly three-quarters of respondents are white and the overall racial makeup of the sample was largely consistent with clinical trial participation nationally for approved drugs. As depicted, proportions of respondents by major age group are fairly evenly distributed with the largest proportions in the 18-34 and 35-44 age groups.

In this use case, respondents indicated their burden of disease from a list of 39 indication areas provided in the survey. For analysis purposes, indications can be aggregated to five broad therapeutic areas. Therapeutic area representation was relatively evenly distributed with nearly a third of respondents specifying that they have autoimmune, metabolic, or central nervous system (CNS) disorders; one quarter have cardiovascular disorders; and 16% have oncology-related disorders.

As depicted, mean procedure group burden scores are largely consistent with the results from the pilot study, though most group scores were lower, as depicted in Table 3.

Table 3

| Category | Average Pilot Study Burden Score | Average New Study Burden Score |
|---|---|---|
| Medication Procedures | - | 47.0 |
| Lab and Blood Test Procedures | 46.4 | 46.0 |

(continued)

| Category | Average Pilot Study Burden Score | Average New Study Burden Score |
|---|---|---|
| Routine Examination Procedures | 41.5 | 40.9 |
| Noninvasive Procedures | 44.3 | 40.7 |
| Invasive Procedures | 75.2 | 70.2 |
| Radiography and Imaging Procedures | 55.4 | 47.2 |
| Questionnaire Procedures | - | 40.8 |
| Additional Requirements | 41.8 | 46.0 |

Routine examinations, noninvasive, and questionnaire procedures are then evaluated as having the lowest burden (e.g., means of 40.9, 40.7, and 40.8 out of 100). Medication, lab and blood test, radiography and imaging, and additional procedures all have similar mean burden scores just below the midpoint rating of 50 (e.g., means of 47.0, 46.0, 47.2, and 46.0). Invasive procedures were rated the most burdensome with a mean of 70.2.

The analytics server may utilize an iterative multivariate elimination regression modeling protocol to determine which key subgroup variables have/had a statistically significant role in predicting participant burden in each procedure group, as depicted in Table 4.

| | Gender | Clinical Participation | Caregiver Reliance | White vs Non-White Race | North American vs Non-North American | Age | Oncology TA | Autoimmune TA | Cardiovascular TA | Metabolic TA | CNS TA |
|---|---|---|---|---|---|---|---|---|---|---|---|
| Medication Burden | | X | X | X | | X | X | X | X | X | X |
| Lab Burden | | X | X | X | | X | X | | X | | |
| Routine Burden | | X | X | X | | X | X | | X | | X |
| Non-Invasive Burden | | X | X | X | | X | X | | | | |
| Invasive Burden | X | | X | X | X | X | | | | | |
| Imaging Burden | | X | X | X | | X | | X | | | X |
| Questionnaire Burden | X | X | X | X | | | X | X | X | | X |
| Additional Burden | | X | X | X | | X | X | | | | |

Table 4

The variables appearing in the highest number of groups include caregiver reliance, which significantly raises predicted model burden scores, and race (as depicted, race might indicate a likelihood of increase or decrease in the overall burden score). The inclusion of individuals who had prior experience as a clinical trial participant may result in predictions of a moderately higher burden score for each procedure group and the inclusion of age predicted that older individuals might rate the burden of each procedure group lower, apart from invasive procedures. The therapeutic area in which the respondent's disease condition belonged is varied in their usefulness. The inclusion of an oncology indication in a model resulted in a higher predicted burden score while the inclusion of any other indication resulted in a lower prediction. Geographic region and participants' sex may also be predictive of participation burden scores in very few procedure group models.

Table 5 details participant perceptions of clinical trial travel and visits. As depicted, the majority (70%) of respondents prefer visits lasting less than 60 minutes. And nearly six-out-of-ten respondents consider four or more visits to be burdensome. The percentage of respondents indicating "somewhat" or "very burdensome" increases depending on the mode of transportation from walking (31.7%) to driving (44.0%), to taking a train (61.0%), to flying (70.4%). Moreover, as depicted, a third of respondents indicated that clinical trial visits would be "somewhat" or "very burdensome" if a portion of the visits could be conducted at home, indicating that most would consider this arrangement to be less burdensome.

Table 5

|  | Percent of Total |
|---|---|
| Typical time willing to spend at each study visit center |  |
| Less than 15 Minutes | 8.9% |
| 15 Minutes to 30 Minutes | 26.1% |
| 30 Minutes to 60 Minutes | 34.5% |
| 1 to 2 Hours | 20.6% |
| 2 to 3 Hours | 5.6% |
| More than 3 Hours | 4.2% |
| Number of visits during a study before it becomes burdensome |  |
| 1-3 Visits | 40.1% |
| 4-6 Visits | 20.9% |
| More than 6 Visits | 39.0% |
| Consider the following somewhat or very burdensome |  |
| You could walk to the study clinic | 31.7% |
| You had to drive yourself to the study clinic | 44.0% |
| You had to travel to the study clinic by train | 61.0% |
| You had to travel to the study clinic by airplane | 70.4% |
| Lab tests and bloodwork conducted at a local clinic | 32.8% |
| Some procedures conducted at the study clinic, but other procedures at a hospital | 50.7% |
| Some procedures conducted at the study clinic, but other procedures at home | 33.6% |
| All procedures conducted at the study clinic | 36.3% |

As described above, the analytics server may execute univariate and multivariate regression modeling to assess different variables to predict a final burden score. The results of univariate regression modeling assessing eight independent variables to predict final patient burden scores are detailed in tables 6 and 7. Sensitivity analyses can be conducted using low, medium, and high variable weighting factors in association with protocol performance outcomes. In six of the eight outcomes, the low weighting approaches are shown to have the highest predictive values, assessed via the univariate model's $R^2$ value. Results are statistically significant for five of the eight low weighting factors in the regression model including those predicting screen failure rate, first patient first visit to last patient first visit (FPFV to LPFV), protocol approval to database lock, percent of trial screening duration to total duration, and number of amendments, with significance at the $p < 0.05$ level. For each single unit of an increase in an independent variable, patient burden is predicted to increase by the coefficient value listed.

Table 6

| Variables | Low Weighting Factors Burden Score $R^2$ | Medium Weighting Factors Burden Score $R^2$ | High Weighting Factors Burden Score $R^2$ |
|---|---|---|---|
| Screen Failure Rate | 6.6% | 7.0% | 6.9% |
| Protocol Approval to FPFV | 0.2% | 0.2% | 0.1% |
| FPFV to LPLV | 2.8% | 2.6% | 2.3% |
| LPLV to DBL | 0.3% | 0.2% | 0% |
| Protocol Approval to DBL | 7.5% | 6.9% | 5.7% |
| Dropout Rate | 0.9% | 0.5% | 0.2% |
| Percent Screening Duration to Total Duration* | 5.3% | 5.4% | 5.2% |
| Protocol Amendments | 4.3% | 4.3% | 4.0% |

In Table 6, the screen failure rate is calculated by dividing a number of screen failures by a number of patients screened. Moreover, the dropout rate is calculated by dividing a number of patients dropped out by a number of patients enrolled. Moreover, the percent screening duration to total duration is calculated by using the following formula:

$$\text{(Last Patient First Visit Date - First Patient First Visit Date) / (Protocol Approval Date – Database Lock Date) * 100.}$$

Table 7

| Variable | Coefficient | 95% Confidence Interval | Standard Error | p-Value |
|---|---|---|---|---|
| Screen Failure Rate (%) | 9568.2 | 7386.4, 5305.6 | 3019.2 | 0.0003 |
| Protocol Approval to FPFV (Days) | 5.8 | -14.0, 25.6 | 10.0 | 0.563 |
| FPFV to LPLV (Days) | 2.9 | 0.4, 5.4 | 1.3 | 0.025 |
| LPLVto DBL (Days) | 8.8 | -21.8, 39.3 | 15.4 | 0.569 |
| Protocol Approval to DBL (Days) | 4.8 | 1.4, 8.3 | 1.7 | 0.006 |
| Dropout Rate (%) | 3040.3 | -2139.9, 8220.4 | 2621.7 | 0.248 |
| Percent Screening Duration to Total Duration (%) | -224.8 | -415.9, -33.7 | 96.3 | 0.022 |
| Protocol Amendments | 1093.0 | 343.0, 1842.8 | 380.1 | 0.005 |

The results discussed herein of this study demonstrate that creation and use of a participant burden score is feasible and associated with at least a selected portion of the protocol performance outcomes. The methods and systems discussed herein expand the scope of variables used to derive an enhanced participation burden score (including participation convenience and lifestyle) and illustrate the predictive validity of a more comprehensive burden score on a larger number of performance outcomes measures. The participation burden algorithm derived using the methods discussed herein are predictive of select study cycle times; screen failure rates; and the number of substantial protocol amendments.

Using the methods and systems described herein the analytics server can conduct more expansive subgroup analyses, to help mitigate bias and increase confidence in the predictive power of the computer model (including the algorithm discussed herein). The algorithm discussed herein may be a predictor of two of the four study cycle time measures including study conduct duration (FPFV to LPLV) and clinical trial duration (from protocol approval to database lock). The algorithm may not be predictive of study start-up (approval to FPFV) and study closeout (LPLV to database lock) cycle times.

Screening duration as a percentage of total study duration may be inversely related to participation burden. This finding suggests that participants perceive a lower burden as more time is spent screening study volunteers relative to the total trial duration. This may be a function of a less demanding participation experience during the treatment period or a more effective expectation setting during the screening period. The algorithm discussed herein may also be used to demonstrate and affirm that the more complex and burdensome the protocol, the higher the screen failure rate; and the higher the number of unplanned and un-budgeted amendments implemented to modify the protocol after it has been finalized.

When analyzing the data using the computer model and algorithms discussed herein, the analytics server may determine that the dropout rate is not significantly predicted by the participant burden score. This may in part be due to the variety of factors contributing to the decision to drop-out of a clinical trial that are not related to participation burden (e.g., serious adverse reactions to the investigational drug and/or perceived lack of investigational drug efficacy). Moreover, the convenience sample the dataset analyzed illustrate a wide variability in drop-out rates per protocol, which may have contributed to the observed result.

Additionally or alternatively, the analytics server may also gather data associated with design variables and disease specific data and analyze said data to achieve a more granular and complex algorithm.

In a non-limiting example, a server (e.g., the analytics server discussed herein) may send a series of questionnaires to a set of patients or participants of a clinical trial. For instance, the patients may log into a website and use various input elements to input their quantified burdens perceived in relation to one or more clinical studies they have previously participated in. The inputs may correspond to the patients' demographic data as well as how they would rate their burden in relation to specific categories associated with each clinical study they have participated in.

The analytics server may then retrieve data associated with the clinical studies themselves. For instance, various operational parameters and their corresponding data can be retrieved. Using the data retrieved, the analytics server may apply the algorithm discussed herein to generate a patient burden score for (at least a part of) the patients who participated in the questionnaire.

The analytics server may then generate a training dataset that includes data associated with the patients (e.g., demographic or other personal data and/or their inputs to the questionnaire, the patient burden score associated with the patient, and data retrieved regarding the clinical studies, such as their operational parameters).

Using the training data, the analytics server may train a computer model. For instance, the analytics server may label the data within the training dataset using the patient burden score. The analytics server may then use a supervised training method to train the computer model. After training, the computer model may be able to ingest data associated with a new clinical study and predict a patient burden score for different future patients. The model may also be able to intake patient data and predict what burden they would experience. For instance, the patient burden score for the new clinical study may be customized for a particular patient based on that patient's attributes (e.g., age, sex, medical condition, distance from the clinical study, and the like). During training, the model may identify an elasticity for relationships that different data points (within the training dataset) have with the patient burden. In that way, the model may determine (and sometimes filter) which features are more important than others.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the spirit or scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope and spirit being indicated by the following claims.

**Claims**

1. A method comprising:

    in response to transmitting a clinical study questionnaire to a set of patients associated with a set of clinical studies, retrieving, by a processor, input received via the set of patients, the input corresponding to demographic data and a quantified burden associated with each clinical study;
    generating, by the processor, a training dataset comprising:

        each patient's demographic data,
        a patient burden score for each patient generated in accordance with an algorithm evaluating each patient's input with regards to participation logistics, lifestyle factors, caregiver involvement, and procedural burden associated with each clinical study, and
        a set of operational parameters associated with the set of clinical study; and

    training, by the processor, a computer model using the training dataset, such that the computer model is configured to ingest data associated with a new clinical study and predict a new patient burden score.

2. The method of claim 1, wherein the new patient burden score is further dependent on an attribute of the new patient.

3. The method of claim 1, further comprising:
    populating, by the processor, at least one graphical user interface using the new patient burden score.

4. The method of claim 1, wherein the set of operational parameters comprises at least one of medications, lab tests, blood tests, examinations, non-invasive procedures, invasive procedure, imaging procedure burden, or self-assessment questionnaire burden.

5. The method of claim 1, wherein the trained computer model identifies an estimated elasticity for a relationship between strength of features within the training dataset.

6. The method of claim 1, wherein training comprises using an iterative multivariate elimination regression modeling protocol to determine which input has a statistically significant relationship with the patient burden score.

7. The method of claim 1, wherein the training dataset is labeled and the computer model is trained via a supervised training method.

8. A system comprising:

    a server comprising a processor and a non-transitory computer-readable medium containing instructions that when executed by the processor causes the processor to perform operations comprising:

        in response to transmitting a clinical study questionnaire to a set of patients associated with a set of clinical studies, retrieve input received via the set of patients, the input corresponding to demographic data and a quantified burden associated with each clinical study;
        generate a training dataset comprising:

            each patient's demographic data,
            a patient burden score for each patient generated in accordance with an algorithm evaluating each patient's input with regards to participation logistics, lifestyle factors, caregiver involvement, and procedural burden associated with each clinical study, and

a set of operational parameters associated with the set of clinical study; and

train a computer model using the training dataset, such that the computer model is configured to ingest data associated with a new clinical study and predict a new patient burden score.

9. The system of claim 8, wherein the new patient burden score is further dependent on an attribute of the new patient.

10. The system of claim 8, wherein the instructions further cause the processor to:
populate at least one graphical user interface using the new patient burden score.

11. The system of claim 8, wherein the set of operational parameters comprises at least one of medications, lab tests, blood tests, examinations, non-invasive procedures, invasive procedure, imaging procedure burden, or self-assessment questionnaire burden.

12. The system of claim 8, wherein the trained computer model identifies an estimated elasticity for a relationship between strength of features within the training dataset.

13. The system of claim 8, wherein training comprises using an iterative multivariate elimination regression modeling protocol to determine which input has a statistically significant relationship with the patient burden score.

14. The system of claim 8, wherein the training dataset is labeled and the computer model is trained via a supervised training method.

15. A system comprising:

a server configured to:

in response to transmitting a clinical study questionnaire to a set of patients associated with a set of clinical studies, retrieve input received via the set of patients, the input corresponding to demographic data and a quantified burden associated with each clinical study;

generate a training dataset comprising:

each patient's demographic data,

a patient burden score for each patient generated in accordance with an algorithm evaluating each patient's input with regards to participation logistics, lifestyle factors, caregiver involvement, and procedural burden associated with each clinical study, and

a set of operational parameters associated with the set of clinical study; and

train a computer model using the training dataset, such that the computer model is configured to ingest data associated with a new clinical study and predict a new patient burden score.

16. The system of claim 15, wherein the new patient burden score is further dependent on an attribute of the new patient.

17. The system of claim 15, wherein the server is further configured to:
populate at least one graphical user interface using the new patient burden score.

18. The system of claim 15, wherein the set of operational parameters comprises at least one of medications, lab tests, blood tests, examinations, non-invasive procedures, invasive procedure, imaging procedure burden, or self-assessment questionnaire burden.

19. The system of claim 15, wherein the trained computer model identifies an estimated elasticity for a relationship between strength of features within the training dataset.

20. The system of claim 15, wherein training comprises using an iterative multivariate elimination regression modeling protocol to determine which input has a statistically significant relationship with the patient burden score.

100

| Processor(s) | Volatile Memory | Communication |
| 105 | 110 | Interface(s) |
| | | 115 |

130

Non-Volatile
Memory
120

Operating
System
135

Application(s)
140

Data
145

User Interface
125

GUI
150

I/O Device(s)
155

# FIG. 1A

160

Client
165A

Client
165B

Client
165N

Network
170

Software 180

Platform 185

Infrastructure 190

Server 195

Cloud
175

170B

# FIG. 1B

200

212 — Identity Provider

165 — Client(s)

Patient Burden Calculation Engine — 202

208 — Gateway Service(s)

Prediction Feeds — 206

210 — SaaS Application(s)

# FIG. 2

**FIG. 3**

**400**

400

| Generate a training dataset including clinical studies and patient data. **402** |
| --- |

↓

| Train a computer model to determine an algorithm corresponding to the patient's quantified burden inputs in relation to the clinical study. **404** |
| --- |

# FIG. 4A

**406**

Learn the structure of the model
- Survey Data (ZS) and their relationship with latent factor as Patient Burden Score

Survey Data | Hypothesis Data

Learning Causal factors at aggregated level to understand sensitivity of segment level features

**408**

Establish relationship between burden scores and op. metrics
- Latent score (PBA) with operational metrics
- Sub-burden scores and how they are influenced by features in protocol design

Operational Data | Protocol Data | PBA Data

xxx | Target Burden Boore

Learning sub-burden factor impact of latent representation by therapy area.

**410**

Estimate elasticity of the relationship between
- Strength of feature and operational metrics
- Effect of therapy on the strength of the relationship

Operational Data | Protocol Data | PBA Data | Other Data Sources

*Illustration*

Overall Importance: Mean Absolute Score

Strength of feature Analysis

**FIG. 4B**

EP 4 220 649 A1

The resulting algorithm was validated through a retrospective exercise to understand the correlation of patient burden with trial performance

**Simplified Algorithm**

A simplified patient burden algorithm was applied to a cohort of 266 clinical protocols

412

**Validation**

The algorithm was used to calculate patient burden scores for each protocol to assess correlation with 8 operational metrics to establish the link

414

**Findings**

Significant correlation was established for 5 of 8 operational metrics:

- Screen Failure Rate
- FPFV to LPLV
- Protocol Approval to Database Lock
- %Screen Duration to Total Duration
- Protocol Amendments
- Protocol Approval to FPFV
- LPLV to Database Lock
- Dropout Rate

Next Steps: Explore findings and expand model development to further clarify variable relationships

414

**FIG. 4C**

| | Individual Characteristics | Burden of Disease Characteristics | Protocol Design Characteristics |
|---|---|---|---|
| Independent Variables | • Demographics<br><br>• Familiarity with clinical research | • Lifestyle restrictions<br><br>• Mobility restrictions<br><br>• Work/School requirements<br><br>• Caregiver involvement | • Procedures<br><br>• Planned visits<br><br>• Virtual Trial Elements |

| | Key performance measures |
|---|---|
| Dependent Variables | • Overall and milestone cycle times<br>• Screening and enrollment effectiveness<br>• Quality (e.g., deviations, substantial amendments) |

**FIG. 4D**

**Algorithm input variables consider procedural, lifestyle, demographics and logistics-based burden assessment**

| Burden score assessment across 57 procedures that can be divided into the following groups: | To calculate patient burden, the algorithm also takes into account lifestyle variables & participation logistics: |
|---|---|

| | | | | Lifestyle Variables | Participation Logistics |
|---|---|---|---|---|---|
| 10 | Routine Procedures | 5 | Non-Invasive Procedures | General Lifestyle Restrictions | Level of Virtual Trial Methodology |
| 6 | Medication Procedures | 11 | Invasive Procedures | Transportation | Average Distance from Clinic |
| 9 | Lab Procedures | 4 | Questionnaire Procedures | Work or School Requirements | Number of Visits & Visit Time |
| 8 | Imaging Procedures | 4 | Additional Procedures | Child Care Arrangements | Patient Pickup Service |
| | | | | Caregiver Status | Procedures Performed in Hospital/Clinic |

**FIG. 4E**

EP 4 220 649 A1

## Baseline Algorithm Derivation and Testing

| Participant Subgroup | Sum of All Protocol Procedure Burden Scores | Weighted by Participation Logistics & Lifestyle Factors |
|---|---|---|
| □ Race/Ethnicity<br>□ Disease Condition<br>□ Age<br>□ Caregiver Reliance<br>□ Past participation | □ Routine<br>□ Medication<br>□ Assessments<br>□ Lab and Imaging<br>□ Questionnaires | □ Willingness to travel<br>□ Visit Frequency<br>□ Location |

**FIG. 4F**

EP 4 220 649 A1

**Europäisches Patentamt**
**European Patent Office**
**Office européen des brevets**

# EUROPEAN SEARCH REPORT

Application Number

EP 23 15 4341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/206521 A1 (WALPOLE KIM MARIE [US] ET AL) 4 July 2019 (2019-07-04) * The whole document, in particular: Paragraphs [0006], [0007], [0037], [0041] – [0047], [0074], [0075]. Figure 10. * ----- | 1-20 | INV. G16H10/20 G06N20/00 |
| | | | TECHNICAL FIELDS SEARCHED (IPC) |
| | | | G16H G06N |

The present search report has been drawn up for all claims

| Place of search | Date of completion of the search | Examiner |
|---|---|---|
| Munich | 11 May 2023 | Zacharias, Malte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 4341

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2023

| Patent document cited in search report | | Publication date | Patent family member(s) | | Publication date |
|---|---|---|---|---|---|
| US 2019206521 | A1 | 04-07-2019 | US 2019206521 | A1 | 04-07-2019 |
| | | | US 2022310216 | A1 | 29-09-2022 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63304844